# EUROPEAN PATENT SPECIFICATION

(11) **EP 0 617 919 B1**
(45) Date of publication and mention of the grant of the patent: **12.06.2002**
(21) Application number: 94104962.9
(22) Date of filing: 29.03.1994
(51) Int. Cl.: A61B 5/117, G06K 9/20, G06K 9/00

(54) **Apparatus for obtaining ridge line pattern image on a curved surface**
Gerät zur Erzielung von Rippenlinenmusterbildern auf einer gekrümmten Oberfläche
Appareil pour obtenir des images de lignes de crêtes sur une surface courbe

(30) Priority: 30.03.1993 JP 7140193
(43) Date of publication of application: 05.10.1994
(73) Proprietor: NEC CORPORATION, Tokyo (JP)
(72) Inventor: Ohta, Naoya, Minato-ku, Tokyo (JP); Hoshino, Yukiko, c/o NEC Security Systems, Ltd., Minato-ku, Tokyo (JP); Shibuya, Tatsuo, c/o NEC Security Systems, Ltd., Minato-ku, Tokyo (JP)
(74) Representative: VOSSIUS & PARTNER

(56) References cited:
- EP-A- 0 194 783
- EP-A- 0 359 554
- WO-A-86/00009
- US-A- 3 906 520
- US-A- 4 120 585
- US-A- 4 783 167
- US-A- 5 096 290

## Description

This invention relates to an apparatus for obtaining ridge line pattern image on a curved surface of an object.

Means for identifying individuals through fingerprint or palm print images has been proposed wherein a fingerprint(or a palm print) image is obtained and is automatically compared with previously stored images using pattern recognition technique.

Heretofore, for obtaining a palm print image, printing ink applied on a surface of a palm is transcribed on a sheet to print a palm picture. A palm print image is obtained from the palm picture by a TV camera or by an image scanner. Although a same method has been used for obtaining a fingerprint image, an apparatus has recently been disclosed for obtaining a fingerprint image without using printing ink. This apparatus which has been disclosed by U. S. Patent Number 5,096,290 entitled "Apparatus for Imaging Fingerprint using Transparent Optical Means having Elastic Material Layer"(hereafter will be called the prior art) may also be used for obtaining a palm print image when a surface for placing an object is broad enough for a palm.

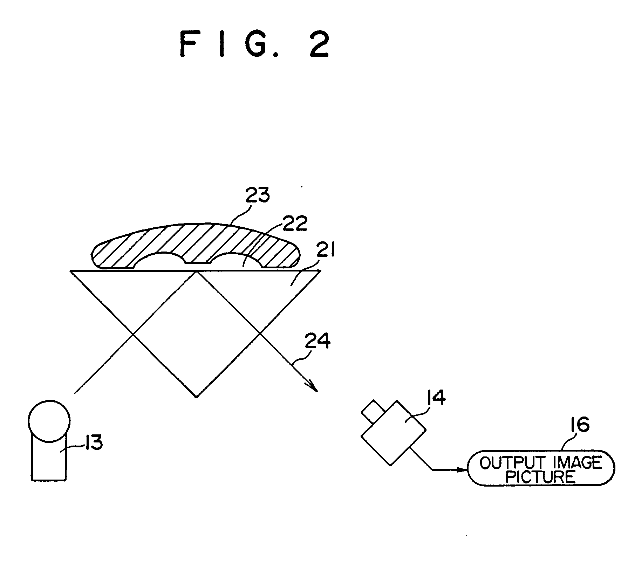

Fig. 2 shows an assembly of the prior art apparatus for obtaining an image pattern of a palm print.

Light beams from a light source 13 incident upon a surface 22 of an optical body 21, are totally reflected at the surface 22 of the body 21, due to the difference of refractive index between the optical body 21 and the surrounding air. When a part of a palm of a hand 23 is in contact with the surface 22, light beams are scattered in a diffused reflection at the contact point. A TV camera 14 is placed at a position for scanning light beams of total reflection and a part of diffused light beams reflected in a direction parallel to the light beams of total reflection, to produce ridge line pattern image of a palm on the surface 22. The surface 22 on which an object is placed in contact is hereafter called a platen.

Poor contact between an object and the platen is a serious problem of the prior art. As is evident from Fig. 2, only a small portion of the total palm area is in contact with the platen 22. When pressure from the hand 23 is increased to increase contact area, the portion which has already been in contact with the platen receives excessive pressure and the ridge lines in the portion can be depressed or the palm itself can be deformed resulting in an inaccurate image pattern.

As an attempt to circumvent this problem, US 3,906,520 discloses a method which employs a fiber optic imaging block having a partially cylindrical, angled objective surface and a common light entry and exit surface.

An object of the invention is to provide means for facilitating compensating position coordinates obtained from a curved surface to coordinates on a two-dimensional plane.

In order to achieve these objects, an apparatus according to claim 1 is provided, wherein the platen surface is made a convex surface which conforms to a concave surface of an object in case when the object is a palm, or the platen surface is made a concave surface which conforms to a convex surface of an object in case when the object is a head of a finger.

It is not necessary to give a rigorous dimension to the curvature of a platen, as objects to be placed on the platen have a good elasticity.

And, as for further coordinates compensation, conventional means can be employed since shape of a platen surface is known.

Further objects, features, and advantages of this invention will become apparent from a consideration of the following description, the appended claims, and the accompanying drawings in which the same numerals indicate the same or the corresponding parts.
Figs. 1a, b show an assembly of a first descriptive embodiment,
Fig. 2 shows an assembly of a prior art,
Fig.3 shows a block diagram of a coordinates compensator of Fig. 1,
Fig. 4 shows data compensated in coordinates compensation,
Fig.5 shows an assembly of a first embodiment of this invention,
Fig. 6 shows an assembly of a second descriptive embodiment,
Fig.7 shows an assembly of a second embodiment of this invention.

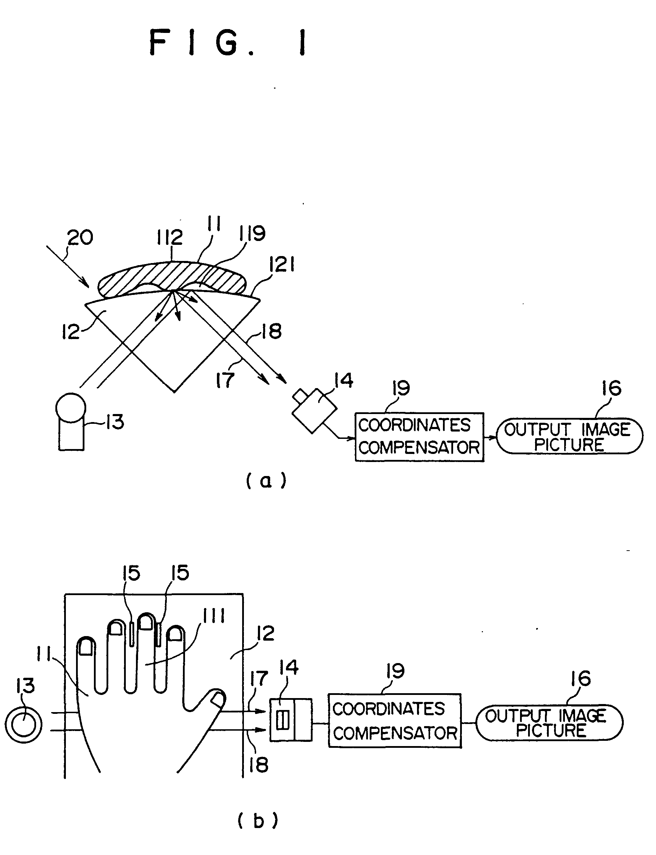

Referring to Figs. 1, there is shown a first descriptive embodiment. Fig. 1a shows an elevation view, and Fig. 1b shows a plan view. On a platen 121 of an optical body 12 is placed a hand 11, the palm of the hand 11 being an object surface of image acquisition. The body 12 is made of such a transparent material as glass or plastic having refractive index larger than air, and the surface of the platen 121 is made a convex surface which fits together with a concave surface of a palm, making a good contact between these two surfaces. The body 12 is a prism, and an advantage of a prism is that an external light beams such as denoted by 20 do not enter a light receiving unit 14.

In order to obtain a reliable contact between the palm and the platen 121, the platen 121 may be coated by transparent elastic film having a same refractive index as the body 12. Method of film coating on the platen will not here be described as it is described in detail in the prior art.

The guide 15 is to define position of the hand 11. The middle finger 111 of the hand is to be placed between the guide 15 when the hand 11 is placed on the platen 121.

As the light receiving unit 14 of Fig. 1, a CCD TV camera for scanning an area is used. A light source 13 illuminates uniformly the whole area scanned by the light receiving unit 14.

In the first descriptive embodiment shown by Fig. 1, the light receiving unit 14 is placed at a position for receiving light beams of total reflection from the platen boundary of the prism 12. It is well known that a total reflection occurs when incident angle of a light beam exceeds a value determined by the refractive index of the material of the prism 12. The light source 13 is placed at a position where all light beams from the light source 13 receive total reflection at the platen boundary of the prism.

When a palm is placed in contact with the platen 121, ridge lines of the palm come in contact with the surface of the platen 121, and grooves between ridge lines do not touch the surface of the platen 121, leaving air space between the platen surface and the palm. A light beam which is incident on a palm body through the platen boundary, receives a diffused reflection, and only a component 17 of light beams reflected in the direction to the light receiving unit 14, is received by the unit 14. A light beam incident on a spot where there is an air space beyond the platen boundary, the light beam receives a total reflection as shown by a light beam 18.

Thus, ridge lines in a palm are converted by the light receiving unit 14, to signals representing dark pixels surrounded by bright pixels.

Extraneous light beams(represented by numeral 20, for example) do not reach the light receiving unit 14, owing to the prismatic shape of the optical body 12.

Output of the light receiving unit 14 is compensated by a coordinates compensator 19, and is stored as an output image picture.

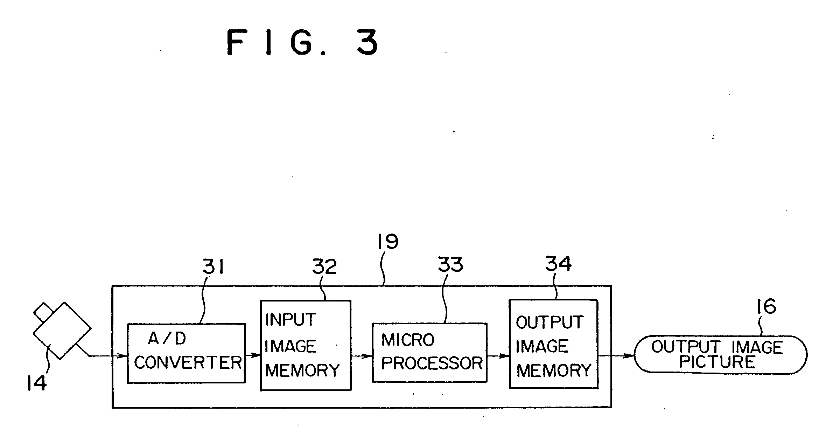

Fig. 3 shows a block diagram of the coordinates compensator 19. The compensator 19 comprises an A/D converter 31 for quantizing signals from the light receiving unit 14, an input image memory 32 for storing output of the A/D converter 31, a micro-processor 33 for processing the compensation, and an output image memory 34 for storing output signals of the processor 33.

Two types of coordinates compensation are necessary in the present invention. An angle of vision from the light receiving unit 14 to the palm, which is shown by the light beam 17, is not perpendicular to the palm surface, and the ridge line pattern of a palm must be displayed as a pattern seen from a direction perpendicular to the palm surface. And by the apparatus of Fig. 1a, a ridge line pattern is obtained on a curved surface of the platen 121, while the ridge line pattern of a palm must be compensated as a pattern on a flat surface. These two types of compensation are performed in the processor 33.

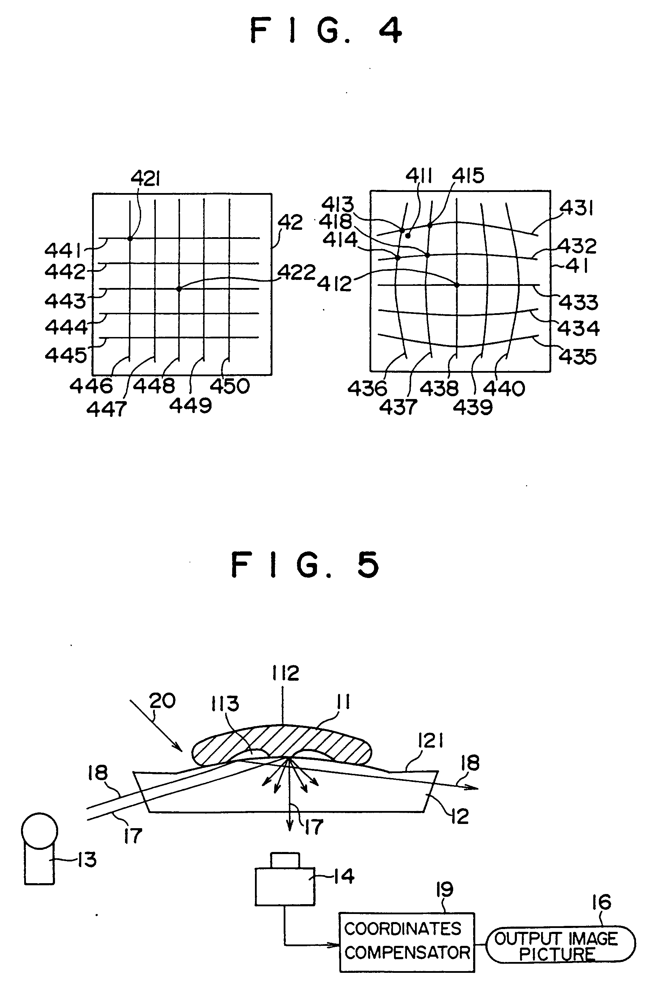

In Fig. 4, a part 41 of the input image memory 32 is shown with its Y-address denoted by 431, 432, •••435, •••, and X-address denoted by 436, 437, •••440, •••. These Y-address and X-address correspond to coordinates positions of pixels displayed on a display. At each cross point of these Y-address and X-address of the input image memory 41, data of a pixel at position corresponding to the address is stored.

On the other hand, a part 42 of the output image memory 34 is shown with its Y-address denoted by 441, 442, •••445, •••, and X-address denoted by 446,447, ••• 450, •••. To each cross point of these Y-address and X-address, a pixel data is to be stored for displaying as the output image picture 16.

Correspondence between the address of the output image memory 34 and the input image memory 32 is easily calculated from the angle of sight of the light receiving unit 14 and from the curvature of the platen 121. A point 412 on the input image memory 41 is the origin of the coordinates. A point 422 on the output image memory 42 is taken as the origin of the coordinates of the memory. A pixel data at the point 412 of the input image memory 41 can be stored as a pixel data at the point 422 of the output image memory 42.

When an address point in the output image memory 42 is transformed to an address point in the input image memory 41, the calculated results have, in general, fractional parts. For example, an address point 421 in the output image memory 42 corresponds to point 411 in the coordinates of the input image memory 41. Pixel data at a point 411 is not stored in the input image memory 32. Therefore, pixel data at the address point 421 of the output image memory 34 is calculated by interpolating four pixel data 413, 414, 415, and 416 of the input image memory 32. Usually, coordinates correspondence is previously calculated and a conversion table for converting address points in the output image memory to coordinates points in the input image memory, is provided in the micro processor 33. The interpolation for calculating pixel data at address points of the output image memory 34 is carried out in the processor 33.

Now, referring to Fig. 5, a first embodiment of this invention is explained. In the first embodiment, the light source 13 is placed at a position where all light beams receive a total reflection at the curved platen surface 121 which is a boundary of the optical body 12, and the light receiving unit 14 is placed at a position where only diffused light beams(indicated by numeral 17, for example) are received and light beams of total reflection(indicated by numeral 18, for example) pass beyond the field of vision of the unit 14. And, in the embodiment shown by Fig. 5, the light receiving unit 14 is placed at a point where an angle of vision from the unit to the platen 121 is a right angle. In a strict terminology, the light receiving unit 14 is placed with its light axis coincident with a normal line to the platen surface at a predetermined point of the platen 121. This position of the light receiving unit 14 can eliminate angle of vision type coordinates compensation in the coordinates compensator 19. But the surface of the optical body 12, which faces to the unit must be a plane, and the extraneous light beam 20 might reach the unit 14 and might deteriorate signal to noise ratio in the output of the light receiving unit 14.

At a point 112 where a ridge line is in contact with the platen 121, a diffused reflection occurs, and at a point 113 where there is an air film between palm surface and the platen 121, a total reflection occurs. Thus ridge lines are converted to groups of bright pixels surrounded by dark pixels representing grooves between ridge lines.

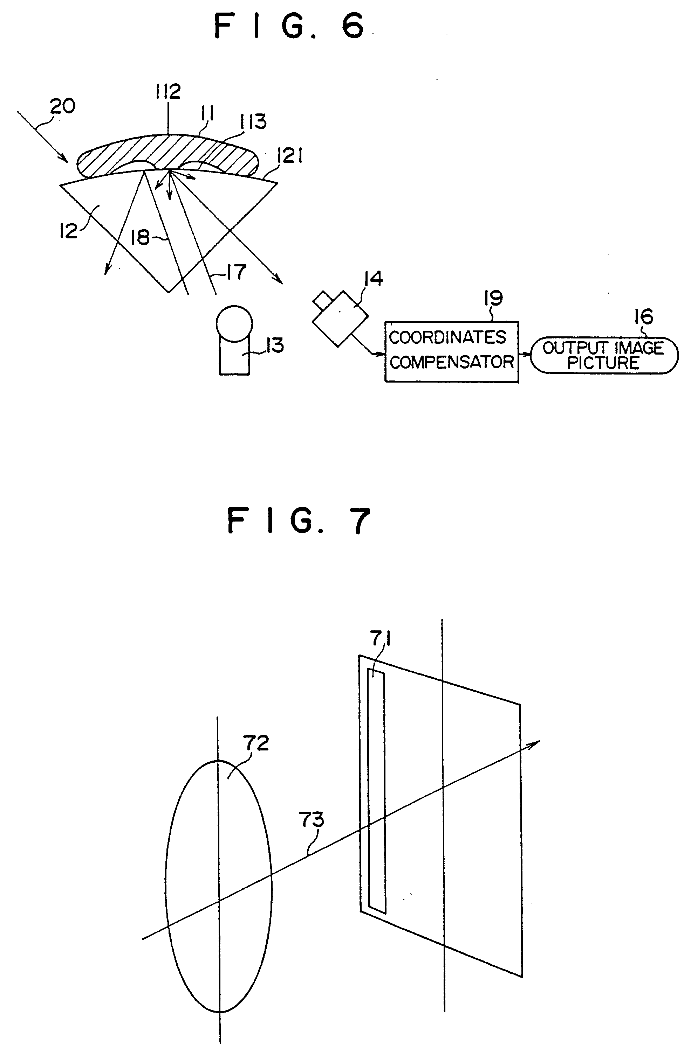

In a second descriptive embodiment as shown in Fig. 6, an optical body 12 is a prism similar to that shown in Figs. 1.

The light source 13 and the light receiving unit 14 are placed facing to a same plane of the prism 12. All light beams(for example, a light beam indicated by numeral 18) incident on a boundary between the prism 12 and air film receive a total reflection. Light beams(for example, a light beam indicated by numeral 17) incident on a point where a ridge line touches the platen surface receive a diffused reflection, and a part of the diffused light beam reach the light receiving unit 14. Thus ridge lines are converted to groups of bright pixels surrounded by dark pixels representing grooves between ridge lines.

In the first and the second descriptive embodiments, and the first embodiment of the invention, a TV camera is used as the light receiving unit 14. In the second embodiment of this invention which is shown in Fig. 7, an image scanner 71 is used as the light receiving unit. A lens 72 focuses light beams from the platen 121 on a plane on which the image scanner 71 scans. A light beam 73 is shown as an example of light beams from the platen 121. Primary scanning is an electronic scanning in an axial direction of the scanner 71, and secondary scanning is a displacement scanning in a direction perpendicular to the axis of the scanner 71 in a plane perpendicular to the light beam 73.

This image scanner 71 of Fig. 7 can be used as the light receiving element 14 of embodiments shown in Fig. 1, Fig. 5, and Fig. 6.

## Claims

1. An apparatus for obtaining ridge line pattern image on a curved surface of an object comprising:
an optical body (12) having a curved platen surface, which fits together with said curved surface of said object for making a good contact between these two curved surfaces,
a light source(13)placed at a point where light beams from said light source receive a total reflection at a boundary between said platen and an air space, and receive a diffused reflection at a boundary between said platen and said object;
a light receiving unit (14) placed at a point where light beams of total reflection do not come in sight of said unit; and
a coordinates compensator (19) for converting pixel positions obtained by said light receiving unit to desired pixel positions in an output image memory,
wherein said optical body (12) has a plane perpendicular to a normal line of said curved platen surface at a predetermined point, and said light receiving unit is placed at a point with its light axis coincident with said normal line.

2. An apparatus for obtaining ridge line pattern image of claim 1, wherein said light receiving unit (14) is a TV camera.

3. An apparatus according to claim 1 or 2,
wherein said light receiving unit is an image scanner (71).

4. An apparatus according to any one of claims 1 to 3,
wherein said curved platen surface is coated by a transparent elastic film having a refractive index same with that of said optical body.

5. An apparatus according to any one of claims 1 to 4,
wherein said curved platen surface has a guide (15) for defining position of said object on said curved platen surface.

## Patentansprüche

1. Vorrichtung zur Gewinnung eines Rippenlinienmusterbildes auf einer gekrümmten Oberfläche eines Objekts, mit:
einem optischen Körper (12) mit einer gekrümmten Plattenfläche, die mit der gekrümmten Oberfläche des Objekts zusammenpaßt, zur Herstellung eines guten Kontakts zwischen diesen beiden gekrümmten Oberflächen,
einer Lichtquelle (13), die an einem Punkt angeordnet ist, wo Lichtstrahlen von der Lichtquelle eine Totalreflexion an einer Grenzfläche zwischen der Platte und einem Luftraum erfahren und an einer Grenzfläche zwischen der Platte und dem Objekt eine Streureflexion erfahren;
einer Lichtaufnahmeeinheit (14), die an einem Punkt angeordnet ist, wo Lichtstrahlen der Totalreflexion nicht in den Sichtbereich der Einheit kommen; und
einem Koordinatenkompensator (19) zum Umrechnen von Pixelpositionen, die von der Lichtaufnahmeeinheit gewonnen werden, in gewünschte Pixelpositionen im Ausgangsbildspeicher,
wobei der optische Körper (12) eine Ebene hat, die senkrecht zu einer senkrechten Linie der gekrümmten Plattenfläche an einem vorbestimmten Punkt ist, und die Lichtaufnahmeeinheit an einem Punkt angeordnet ist, wo ihre Lichtachse mit der senkrechten Linie übereinstimmt.

2. Vorrichtung zur Gewinnung eines Rippenlinienmusterbildes nach Anspruch 1, wobei die Lichtaufnahmeeinheit (14) eine Fernsehkamera ist.

3. Vorrichtung nach Anspruch 1 oder 2, wobei die Lichtaufnahmeeinheit ein Bildscanner (71) ist.

4. Vorrichtung nach einem der Ansprüche 1 bis 3, wobei die gekrümmte Plattenfläche mit einem transparenten elastischen Film mit einem Brechungsindex beschichtet ist, der der gleiche ist wie der des optischen Körpers.

5. Vorrichtung nach einem der Ansprüche 1 bis 4, wobei die gekrümmte Plattenfläche eine Führung (15) zur Begrenzung einer Position des Objekts auf der gekrümmten Plattenfläche hat.

## Revendications

1. Appareil pour obtenir une image de motifs de lignes de crêtes sur une surface courbe d'un objet comprenant :
un corps optique (12) ayant une surface de plateau courbe, qui s'ajuste avec ladite surface courbe dudit objet pour créer un bon contact entre ces deux surfaces courbes,
une source lumineuse (13) placée en un point où des rayons lumineux de ladite source lumineuse reçoivent une réflexion totale à une frontière entre le dit plateau et un espace d'air, et reçoivent une réflexion diffuse à une frontière entre ledit plateau et ledit objet ;
une unité réceptrice de lumière (14) placée en un point où les rayons lumineux de réflexion totale ne parviennent pas en vue de ladite unité ; et
un compensateur de coordonnées (19) pour convertir des positions de pixels obtenues par ladite unité réceptrice de lumière en des positions de pixels désirées dans une mémoire d'image de sortie,
dans lequel ledit corps optique (12) a un plan perpendiculaire à une ligne normale de ladite surface de plateau courbe en un point prédéterminé, et ladite unité réceptrice de lumière est placée en un point tel que son axe de lumière coïncide avec ladite ligne normale.

2. Appareil pour obtenir une image de motifs de lignes de crêtes selon la revendication 1, dans lequel ladite unité réceptrice de lumière (14) est une caméra de télévision.

3. Appareil selon la revendication 1 ou 2, dans lequel ladite unité réceptrice de lumière (14) est un scanner d'image (71).

4. Appareil selon l'une quelconque des revendications 1 à 3, dans lequel ladite surface de plateau courbe est recouverte d'un film élastique transparent ayant un indice de réfraction identique à celui dudit corps optique.

5. Appareil selon l'une quelconque des revendications 1 à 4, dans lequel ladite surface de plateau courbe a un guide (15) pour définir la position dudit objet sur ladite surface de plateau courbe.
